# EUROPEAN PATENT APPLICATION

(11) **EP 3 437 651 A1**
(43) Date of publication of application: **06.02.2019**
(21) Application number: 17306037.7
(22) Date of filing: 03.08.2017
(51) Int. Cl.: A61K 38/45, A61K 38/08, A61K 38/10, A61K 38/16, A61P 1/16, C12N 9/12

(54) **PEPTIDES FOR TREATMENT AND PREVENTION OF NONALCOHOLIC FATTY LIVER DISEASE**

(71) Applicant: Université de Strasbourg, 67000 Strasbourg (FR); Institut National de la Santé et de la Recherche Médicale, 75013 Paris (FR)
(72) Inventor: MARION, Vincent, 67640 LIPSHEIM (FR)
(74) Representative: Cabinet Becker et Associés

(57) **Abstract**

The present invention relates to peptides for the treatment or prevention of nonalcoholic fatty liver disease (NAFLD), non-alcoholic fatty liver (NAFL), non-alcoholic steatohepatitis (NASH), hepatic steatosis (fatty liver), liver fibrosis, liver inflammation, cirrhosis, or hepatocellular carcinoma.

SEQUENCE LISTING

<110>  Université de Strasbourg et al

<120>  PEPTIDES FOR TREATMENT AND PREVENTION OF NONALCOHOLIC FATTY LIVER
        DISEASE

<130>  B2558EP

<160>  34

<170>  PatentIn version 3.5

<210>  1
<211>  672
<212>  PRT
<213>  Homo sapiens

<400>  1

Met Ala Asp Val Phe Pro Gly Asn Asp Ser Thr Ala Ser Gln Asp Val
1               5                   10                  15

Ala Asn Arg Phe Ala Arg Lys Gly Ala Leu Arg Gln Lys Asn Val His
            20                  25                  30

Glu Val Lys Asp His Lys Phe Ile Ala Arg Phe Phe Lys Gln Pro Thr
        35                  40                  45

Phe Cys Ser His Cys Thr Asp Phe Ile Trp Gly Phe Gly Lys Gln Gly
    50                  55                  60

Phe Gln Cys Gln Val Cys Cys Phe Val Val His Lys Arg Cys His Glu
65                  70                  75                  80

Phe Val Thr Phe Ser Cys Pro Gly Ala Asp Lys Gly Pro Asp Thr Asp
                85                  90                  95

Asp Pro Arg Ser Lys His Lys Phe Lys Ile His Thr Tyr Gly Ser Pro
            100                 105                 110

Thr Phe Cys Asp His Cys Gly Ser Leu Leu Tyr Gly Leu Ile His Gln
        115                 120                 125

Gly Met Lys Cys Asp Thr Cys Asp Met Asn Val His Lys Gln Cys Val
    130                 135                 140

Ile Asn Val Pro Ser Leu Cys Gly Met Asp His Thr Glu Lys Arg Gly
145                 150                 155                 160

Arg Ile Tyr Leu Lys Ala Glu Val Ala Asp Glu Lys Leu His Val Thr
                165                 170                 175

Val Arg Asp Ala Lys Asn Leu Ile Pro Met Asp Pro Asn Gly Leu Ser
        180                 185                 190

Asp Pro Tyr Val Lys Leu Lys Leu Ile Pro Asp Pro Lys Asn Glu Ser
    195                 200                 205

Lys Gln Lys Thr Lys Thr Ile Arg Ser Thr Leu Asn Pro Gln Trp Asn
    210                 215                 220

Glu Ser Phe Thr Phe Lys Leu Lys Pro Ser Asp Lys Asp Arg Arg Leu
225                 230                 235                 240

Ser Val Glu Ile Trp Asp Trp Asp Arg Thr Thr Arg Asn Asp Phe Met
                245                 250                 255

Gly Ser Leu Ser Phe Gly Val Ser Glu Leu Met Lys Met Pro Ala Ser
            260                 265                 270

Gly Trp Tyr Lys Leu Leu Asn Gln Glu Glu Gly Glu Tyr Tyr Asn Val
        275                 280                 285

Pro Ile Pro Glu Gly Asp Glu Glu Gly Asn Met Glu Leu Arg Gln Lys
    290                 295                 300

Phe Glu Lys Ala Lys Leu Gly Pro Ala Gly Asn Lys Val Ile Ser Pro
305                 310                 315                 320

Ser Glu Asp Arg Lys Gln Pro Ser Asn Asn Leu Asp Arg Val Lys Leu
                325                 330                 335

Thr Asp Phe Asn Phe Leu Met Val Leu Gly Lys Gly Ser Phe Gly Lys
            340                 345                 350

Val Met Leu Ala Asp Arg Lys Gly Thr Glu Glu Leu Tyr Ala Ile Lys
        355                 360                 365

Ile Leu Lys Lys Asp Val Val Ile Gln Asp Asp Asp Val Glu Cys Thr
    370                 375                 380

Met Val Glu Lys Arg Val Leu Ala Leu Leu Asp Lys Pro Pro Phe Leu
385                 390                 395                 400

Thr Gln Leu His Ser Cys Phe Gln Thr Val Asp Arg Leu Tyr Phe Val
                405                 410                 415

Met Glu Tyr Val Asn Gly Gly Asp Leu Met Tyr His Ile Gln Gln Val
            420                 425                 430

Gly Lys Phe Lys Glu Pro Gln Ala Val Phe Tyr Ala Ala Glu Ile Ser
        435                 440                 445

Ile Gly Leu Phe Phe Leu His Lys Arg Gly Ile Ile Tyr Arg Asp Leu
    450                 455                 460

Lys Leu Asp Asn Val Met Leu Asp Ser Glu Gly His Ile Lys Ile Ala
465                 470                 475                 480

Asp Phe Gly Met Cys Lys Glu His Met Met Asp Gly Val Thr Thr Arg
                485                 490                 495

Thr Phe Cys Gly Thr Pro Asp Tyr Ile Ala Pro Glu Ile Ile Ala Tyr
            500                 505                 510

Gln Pro Tyr Gly Lys Ser Val Asp Trp Trp Ala Tyr Gly Val Leu Leu
        515                 520                 525

Tyr Glu Met Leu Ala Gly Gln Pro Pro Phe Asp Gly Glu Asp Glu Asp
    530                 535                 540

Glu Leu Phe Gln Ser Ile Met Glu His Asn Val Ser Tyr Pro Lys Ser
545                 550                 555                 560

Leu Ser Lys Glu Ala Val Ser Ile Cys Lys Gly Leu Met Thr Lys His
                565                 570                 575

Pro Ala Lys Arg Leu Gly Cys Gly Pro Glu Gly Glu Arg Asp Val Arg
            580                 585                 590

Glu His Ala Phe Phe Arg Arg Ile Asp Trp Glu Lys Leu Glu Asn Arg
        595                 600                 605

Glu Ile Gln Pro Pro Phe Lys Pro Lys Val Cys Gly Lys Gly Ala Glu
    610                 615                 620

Asn Phe Asp Lys Phe Phe Thr Arg Gly Gln Pro Val Leu Thr Pro Pro
625                 630                 635                 640

Asp Gln Leu Val Ile Ala Asn Ile Asp Gln Ser Asp Phe Glu Gly Phe
                645                 650                 655

Ser Tyr Val Asn Pro Gln Phe Val His Pro Ile Leu Gln Ser Ala Val
            660                 665                 670

<210>  2

<211>  13
<212>  PRT
<213>  Homo sapiens

<400>  2

Met Val Glu Lys Arg Val Leu Ala Leu Leu Asp Lys Pro
1               5                   10


<210>  3
<211>  20
<212>  PRT
<213>  Homo sapiens

<400>  3

Pro Phe Leu Thr Gln Leu His Ser Cys Phe Gln Thr Val Asp Arg Leu
1               5                   10                  15


Tyr Phe Val Met
            20


<210>  4
<211>  27
<212>  PRT
<213>  Homo sapiens

<400>  4

Arg Leu Tyr Phe Val Met Glu Tyr Val Asn Gly Gly Asp Leu Met Tyr
1               5                   10                  15


His Ile Gln Gln Val Gly Lys Phe Lys Glu Pro
            20                  25


<210>  5
<211>  32
<212>  PRT
<213>  Homo sapiens

<400>  5

Met Tyr His Ile Gln Gln Val Gly Lys Phe Lys Glu Pro Gln Ala Val
1               5                   10                  15


Phe Tyr Ala Ala Glu Ile Ser Ile Gly Leu Phe Phe Leu His Lys Arg
            20                  25                  30


<210>  6
<211>  33
<212>  PRT
<213>  Homo sapiens

<400>  6

Pro Gln Ala Val Phe Tyr Ala Ala Glu Ile Ser Ile Gly Leu Phe Phe

1               5                   10                  15

Leu His Lys Arg Gly Ile Ile Tyr Arg Asp Leu Lys Leu Asp Asn Val
            20                  25                  30

Met

<210>  7
<211>  13
<212>  PRT
<213>  Homo sapiens

<400>  7

Met Asp Gly Val Thr Thr Arg Thr Phe Cys Gly Thr Pro
1               5                   10

<210>  8
<211>  8
<212>  PRT
<213>  Homo sapiens

<400>  8

Met Thr Lys His Pro Ala Lys Arg
1               5

<210>  9
<211>  25
<212>  PRT
<213>  Homo sapiens

<400>  9

Val Glu Cys Thr Met Val Glu Lys Arg Val Leu Ala Leu Leu Asp Lys
1               5                   10                  15

Pro Pro Phe Leu Thr Gln Leu His Ser
            20                  25

<210>  10
<211>  20
<212>  PRT
<213>  Homo sapiens

<400>  10

Met Cys Lys Glu His Met Met Asp Gly Val Thr Thr Arg Thr Phe Cys
1               5                   10                  15

Gly Thr Pro Asp
            20

<210>  11
<211>  21
<212>  PRT
<213>  Homo sapiens

<400>  11

Ser Ile Cys Lys Gly Leu Met Thr Lys His Pro Ala Lys Arg Leu Gly
1               5                   10                  15

Cys Gly Pro Glu Gly
            20

<210>  12
<211>  40
<212>  PRT
<213>  Homo sapiens

<400>  12

Arg Lys Gly Thr Glu Glu Leu Tyr Ala Ile Lys Ile Leu Lys Lys Asp
1               5                   10                  15

Val Val Ile Gln Asp Asp Asp Val Glu Cys Thr Met Val Glu Lys Arg
            20                  25                  30

Val Leu Ala Leu Leu Asp Lys Pro
        35                  40

<210>  13
<211>  29
<212>  PRT
<213>  Homo sapiens

<400>  13

Arg Val Leu Ala Leu Leu Asp Lys Pro Pro Phe Leu Thr Gln Leu His
1               5                   10                  15

Ser Cys Phe Gln Thr Val Asp Arg Leu Tyr Phe Val Met
            20                  25

<210>  14
<211>  36
<212>  PRT
<213>  Homo sapiens

<400>  14

Arg Thr Phe Cys Gly Thr Pro Asp Tyr Ile Ala Pro Glu Ile Ile Ala
1               5                   10                  15

Tyr Gln Pro Tyr Gly Lys Ser Val Asp Trp Trp Ala Tyr Gly Val Leu
            20                  25                  30

Leu Tyr Glu Met
        35

<210>  15
<211>  8
<212>  PRT
<213>  Artificial Sequence

<220>
<223>  PTD R8

<400>  15

Arg Arg Arg Arg Arg Arg Arg Arg
1               5

<210>  16
<211>  15
<212>  PRT
<213>  Artificial Sequence

<220>
<223>  PTD

<400>  16

Arg Arg Pro Arg Arg Pro Arg Arg Pro Arg Arg Pro Arg Arg Pro
1               5                   10                  15

<210>  17
<211>  16
<212>  PRT
<213>  Artificial Sequence

<220>
<223>  PTD

<400>  17

Arg Gln Ile Lys Ile Trp Phe Gln Asn Arg Arg Met Lys Trp Lys Lys
1               5                   10                  15

<210>  18
<211>  11
<212>  PRT
<213>  Artificial Sequence

<220>
<223>  PTD

<400>  18

Tyr Gly Arg Lys Lys Arg Arg Gln Arg Arg Arg
1               5                   10

<210>  19
<211>  25

<212>  PRT
<213>  artificial sequence

<220>
<223>  Stapled peptide sequence A


<220>
<221>  MISC_FEATURE
<222>  (6)..(6)
<223>  Xaa = 2 - (4-pentenyl) alanine

<220>
<221>  MISC_FEATURE
<222>  (13)..(13)
<223>  Xaa = 2 - (4-pentenyl) alanine

<400>  19

Val Glu Cys Thr Met Xaa Glu Lys Arg Val Leu Ala Xaa Leu Asp Lys
1               5                   10                  15


Pro Pro Phe Leu Thr Gln Leu His Ser
            20                  25


<210>  20
<211>  21
<212>  PRT
<213>  artificial sequence

<220>
<223>  Stapled peptide sequence B


<220>
<221>  MISC_FEATURE
<222>  (2)..(2)
<223>  Xaa = 2 - (4-pentenyl) alanine

<220>
<221>  MISC_FEATURE
<222>  (9)..(9)
<223>  Xaa = 2 - (4-pentenyl) alanine

<400>  20

Ser Xaa Cys Lys Gly Leu Met Thr Xaa His Pro Ala Lys Arg Leu Gly
1               5                   10                  15


Cys Gly Pro Glu Gly
            20


<210>  21
<211>  26
<212>  PRT
<213>  artificial sequence

<220>
<223>  Scrambled peptide sequence A

<400>  21

Lys Glu Val Pro Val Asp Thr Cys His Leu Thr Leu Met Leu Leu Phe
1               5                   10                  15

Arg Ser Val Ala Leu Lys Gln His Pro Glu
            20                  25

<210>  22
<211>  21
<212>  PRT
<213>  artificial sequence

<220>
<223>  Scrambled peptide sequence B

<400>  22

Ser Ala Glu Cys Lys Gly Arg His Gly Thr Pro Pro Gly Lys Leu Met
1               5                   10                  15

Ile Cys Lys Gly Leu
            20

<210>  23
<211>  20
<212>  DNA
<213>  artificial sequence

<220>
<223>  primer

<400>  23
tgctttggtt tctgggactt                                                   20

<210>  24
<211>  20
<212>  DNA
<213>  artificial sequence

<220>
<223>  primer

<400>  24
gctctagccg aacacgaatc                                                   20

<210>  25
<211>  22
<212>  DNA
<213>  artificial sequence

<220>
<223>  primer

<400>  25
tggacaaagt agacggagtg tc                                                22

<210>  26
<211>  20
<212>  DNA
<213>  artificial sequence

<220>
<223>  primer

<400>  26
tagcaaggcc tgtcccatac                                                   20


<210>  27
<211>  20
<212>  DNA
<213>  artificial sequence

<220>
<223>  primer

<400>  27
tgagaacttg ccaccgtatg                                                   20


<210>  28
<211>  20
<212>  DNA
<213>  artificial sequence

<220>
<223>  primer

<400>  28
ggcaggtagg cccctatatc                                                   20


<210>  29
<211>  20
<212>  DNA
<213>  artificial sequence

<220>
<223>  primer

<400>  29
gtttcatccg ggtcttcatc                                                   20


<210>  30
<211>  20
<212>  DNA
<213>  artificial sequence

<220>
<223>  primer

<400>  30
gtgtctgtgc cctcgaaaat                                                   20


<210>  31

<211>  20
<212>  DNA
<213>  artificial sequence

<220>
<223>  primer

<400>  31
aagttctctg cctcccgatt                                                   20


<210>  32
<211>  20
<212>  DNA
<213>  artificial sequence

<220>
<223>  primer

<400>  32
caaagcgttg ctggaagttt                                                   20


<210>  33
<211>  20
<212>  DNA
<213>  artificial sequence

<220>
<223>  primer

<400>  33
tcgattccct cctacactgc                                                   20


<210>  34
<211>  20
<212>  DNA
<213>  artificial sequence

<220>
<223>  primer

<400>  34
ttggtggtac tggctcatca                                                   20

## Description

### FIELD OF THE INVENTION

The present invention relates to the field of the medicine. More particularly, it relates to treatment of hepatic steatosis, especially non-alcoholic steatotic hepatitis

### BACKGROUND OF THE INVENTION

NAFLD (nonalcoholic fatty liver disease), defined by the presence of hepatic accumulation of triglycerides in the hepatocytes in absence of any other etiology of liver disease, is the most common cause of chronic liver disease in the Western world. Its clinical-histologic phenotype extends from nonalcoholic fatty liver (NAFL) to nonalcoholic steatohepatitis (NASH), characterized by liver inflammation and progressive fibrosis, leading to cirrhosis and end stage liver disease as well as hepatocellular carcinoma.

Whereas the estimated prevalence of NAFLD ranges from 6 to 33 % in the general population, the prevalence of NASH only ranges from 3 to 5 %, but NASH-related cirrhosis has become the second leading indication for liver transplantation in the United States. Hospitalizations for NAFLD have increased by 97 % since the year 2000.

There are no drugs currently approved to prevent or treat NAFLD or NASH.

Therefore, there is a need for new treatments for preventing or treating nonalcoholic fatty liver disease (NAFLD), non-alcoholic steatohepatitis (NASH), and hepatic steatosis (fatty liver).

### SUMMARY OF THE INVENTION

Surprisingly, the inventors provide a peptide or a combination of peptides which specifically decreases the expression of Solute Carrier Family 27 Member 2 (SLC27A2) commonly known as FATP2 (Fatty acid transport protein 2) in adipose tissue. The peptide or combination of peptides is capable, after 3 months of a single injection, of decreasing the phenomenon of steatosis on the liver, in particular capable of decreasing the size of the lipid droplets in the liver, the level of two biomarkers of liver damage (i.e., AST and ALT) and the ratio of liver weight to body weight.

Accordingly, the present invention relates to a peptide for use in the treatment or prevention of a disease selected from the group consisting of nonalcoholic fatty liver disease (NAFLD), non-alcoholic fatty liver (NAFL), non-alcoholic steatohepatitis (NASH), hepatic steatosis (fatty liver), liver fibrosis, liver inflammation, cirrhosis, or hepatocellular carcinoma.

Preferably, the peptide has the following features:
- the peptide modifies the expression levels of the FATPs expression in adipose tissue, preferentially it decreases the FATP2 expression in adipose tissue;
- the peptide sequence comprises the sequence of a segment of a PKC (Protein Kinase C);
- the peptide comprises at least one methionine, one proline and one arginine;
- the peptide has a length of at least 8 amino acids and less than 40 amino acids, preferably a length of at least 10 amino acids and less than 30 amino acids, more preferably of at least 12 amino acids and less than 25 amino acids; and
- the peptide sequence may comprise 1, 2, 3, 4, or 5 substitution(s), deletion(s), addition(s), or a mixture thereof within said sequence of a segment of a PKC.

In one embodiment, the peptide adopts an alpha helical conformation.

In another embodiment, the peptide is modified by a chemical cross-linking process such as stapling.

In still another embodiment, the peptide sequence comprises a sequence according to one of the following formulae: M-(X)m-P-(X)n-R or M-(X)m-R-(X)n-P or P-(X)m-M-(X)n-R or P-(X)m-R-(X)n-M or R-(X)m-P-(X)n-M or R-(X)m-M-(X)n-P, preferably M-(X)m-R-(X)n-P or P-(X)m-R-(X)n-M or R-(X)m-M-(X)n-P or M-(X)m-P-(X)n-R. In these sequences, X is any amino acid, m and n are integers from 1 to 20, preferably from 2 to 15, even more preferably from 2 to 7, and m + n is less than 30, preferably m + n is less than 20.

In one embodiment, said PKC is selected from the group consisting of an alpha-PKC (αPKC), a beta-PKC (βPKC) including βI and βII PKC, delta-PKC, theta-PKC, eta-PKC and epsilon-PKC, more preferably an αPKC of SEQ ID No 1.

In another embodiment, the peptide sequence comprises a sequence selected from the group consisting of MVEKRVLALLDKP (SEQ ID No 2), PFLTQLHSCFQTVDRLYFVM (SEQ ID No 3), RLYFVMEYVNGGDLMYHIQQVGKFKEP (SEQ ID No 4), MYHIQQVGKFKEPQAVFYAAEISIGLFFLHKR (SEQ ID No 5), PQAVFYAAEISIGLFFLHKRGIIYRDLKLDNVM (SEQ ID No 6), MDGVTTRTFCGTP (SEQ ID No 7), and MTKHPAKR (SEQ ID No 8), preferably from the group consisting of MVEKRVLALLDKP (SEQ ID No 2), PFLTQLHSCFQTVDRLYFVM (SEQ ID No 3), PQAVFYAAEISIGLFFLHKRGIIYRDLKLDNVM (SEQ ID No 6), MDGVTTRTFCGTP (SEQ ID No 7), and MTKHPAKR (SEQ ID No 8), even more preferably from the group consisting of MVEKRVLALLDKP (SEQ ID No 2), MDGVTTRTFCGTP (SEQ ID No 7), and MTKHPAKR (SEQ ID No 8). Optionally, the peptide comprises 1, 2, 3, 4, or 5 substitution(s), deletion(s), addition(s), or a mixture thereof, preferably 1, 2, or 3 substitution(s).

In yet another embodiment, the peptide sequence comprises a sequence selected from the group consisting of VECTMVEKRVLALLDKPPFLTQLHS (SEQ ID No 9), MCKEHMMDGVTTRTFCGTPD (SEQ ID No 10), and SICKGLMTKHPAKRLGCGPEG (SEQ ID No 11), and optionally it comprises 1, 2, 3, 4, or 5 substitution(s), deletion(s), addition(s), or a mixture thereof, preferably 1,2, or 3 substitution(s). In particular, the peptide sequence may consist in a sequence selected from the group consisting of VECTMVEKRVLALLDKPPFLTQLHS (SEQ ID No 9), MCKEHMMDGVTT-RTFCGTPD (SEQ ID No 10), and SICKGLMTKHPAKRLGCGPEG (SEQ ID No 11).

In still another embodiment, the peptide(s) of the present disclosure are modified by a chemical cross-linking process such as stapling, preferably the stapled peptide sequence consists of a sequence selected from the group consisting of VECTM**V**EKRVLA**L**LDKPPFLTQLHS (SEQ ID No 9), MC**K**EHMMDG**V**TTRTFCGTPD (SEQ ID No 10), and S**I**CKGLMT**K**HPAKRLGCGPEG (SEQ ID No 11), wherein the residues which are bold and underlined carry the stapling.

Preferably, the disease is selected from the group consisting of nonalcoholic fatty liver disease (NAFLD), non-alcoholic fatty liver (NAFL), non-alcoholic steatohepatitis (NASH), and hepatic steatosis (fatty liver). More preferably, the disease is hepatic steatosis (fatty liver) or non-alcoholic steatohepatitis (NASH). Still more preferably, the disease is non-alcoholic steatohepatitis (NASH).

In one embodiment, the peptide is used in combination with another drug.

### BRIEF DESCRIPTION OF THE DRAWINGS

**Figure 1****: PATAD's long-lasting effect in preventing hyperglycemia**
   Fig 1A:Time points series in days at 30 minutes glucose bolus in fasting male diet induced obese (DIO) (black line) and controls (dotted line) after a single injection of the PATAD treatment at day 0.
   Fig 1B: Time points series in days at 120 minutes glucose bolus in fasting male diet induced obese (DIO) (black line) and controls (dotted line) after a single injection of the PATAD treatment at day 0. n = 8 animals per group
**Figure 2****: Effect of adipose tissue targeted PATAD treatment on expression levels of key fatty acids transporters and receptors**
   n = 4 animals per group, GAPDH was used as reference gene. There are 6 isoforms of FATP (FATP1-6 also known as SLC27A1-SLC27A6).
   Fatp1 (Fatty acid transport protein 1), Fatp2 (Fatty acid transport protein 2), Fatp3 (Fatty acid transport protein 3), Fatp4 (Fatty acid transport protein 4), Fatp5 (Fatty acid transport protein 5), Fatp6 (Fatty acid transport protein 6), FFAR1 (Free Fatty Acid Receptor 1), FFAR2 (Free Fatty Acid Receptor 2), FFAR3 (Free Fatty Acid Receptor 3), FFAR4 (Free Fatty Acid Receptor 4) "Scramble less" refers to the combination of the two peptides with the same amino acid residues as in the stapled forms but randomly rearranged with a maintained alpha helix structure: Scrambled peptide sequence A and Scrambled peptide sequence B. "Staple" refers to a combination of two stapled peptides: Stapled peptide sequence A and Stapled peptide sequence B.

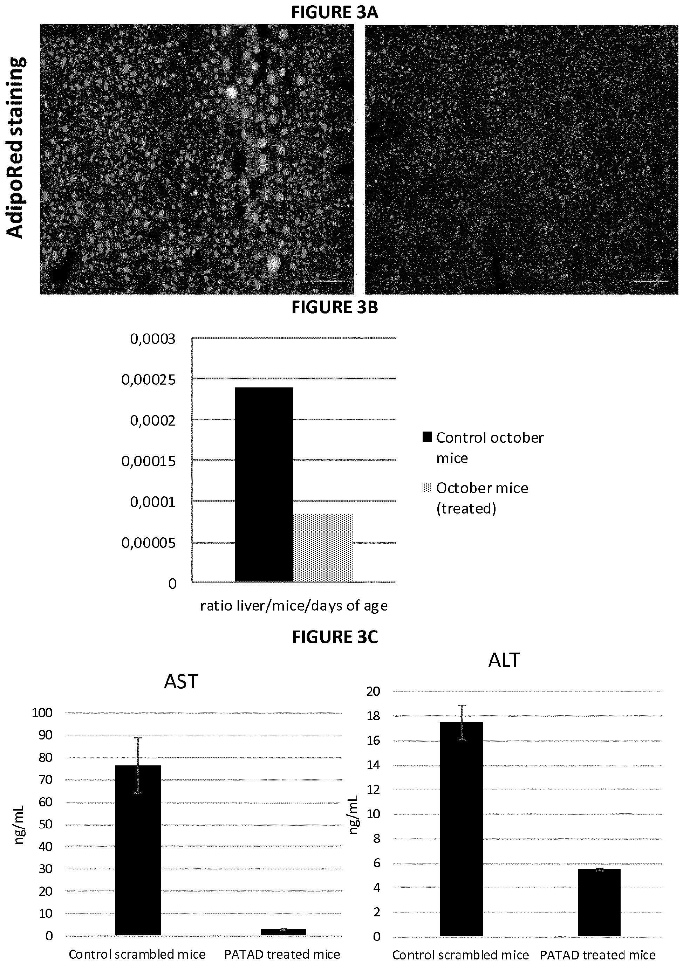

**Figure 3****: Effect on liver after 3 months of adipose tissue targeted PATAD treatment**
   (A) Fluorescent staining of cryosections from fixated liver from control (left) and PATAD (right) treated mice 3 months after one unique PATAD injection.
   (B) Ratio of liver versus body weight times age in days was plotted for n = 1 animal per group.
   (C) Mean values of both AST and ALT measured by ELISA approach in plasma of mice with the indicated treatment. n = 4 samples per group.

### DETAILED DESCRIPTION OF THE INVENTION

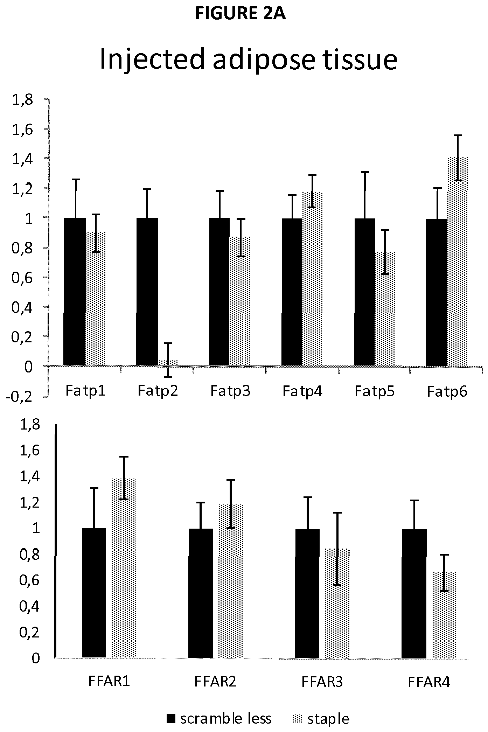

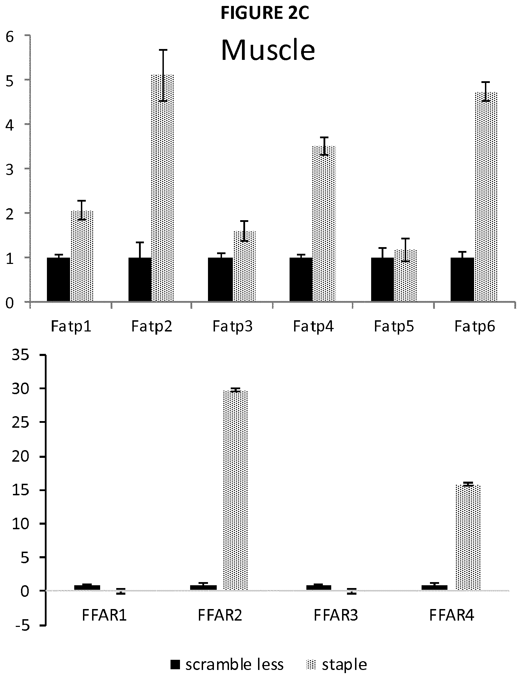

Surprisingly, the inventors provide a peptide or a combination of peptides which specifically decreases the expression of FATP2 (Fatty acid transport protein 2) in adipose tissue (Figure 2A). The peptide or combination of peptides is capable, after 3 months of a single injection, of decreasing the phenomenon of steatosis on the liver, in particular capable of decreasing the size of the lipid droplets in the liver, the level of two biomarkers of liver damage (i.e., AST and ALT) and the ratio of liver weight to body weight (Figure 3).

Accordingly, the invention relates to
- a peptide for use in the treatment or prevention of a disease selected from the group consisting of nonalcoholic fatty liver disease (NAFLD), non-alcoholic fatty liver (NAFL), non-alcoholic steatohepatitis (NASH), hepatic steatosis (fatty liver), liver fibrosis, liver inflammation, cirrhosis, or hepatocellular carcinoma;
- the use of a peptide for the manufacture of a medicine for the treatment or prevention of a disease selected from the group consisting of nonalcoholic fatty liver disease (NAFLD), non-alcoholic fatty liver (NAFL), non-alcoholic steatohepatitis (NASH), hepatic steatosis (fatty liver), liver fibrosis, liver inflammation, cirrhosis, or hepatocellular carcinoma;
- a method for the treatment or prevention of a disease selected from the group consisting of nonalcoholic fatty liver disease (NAFLD), non-alcoholic fatty liver (NAFL), non-alcoholic steatohepatitis (NASH), hepatic steatosis (fatty liver), liver fibrosis, liver inflammation, cirrhosis, or hepatocellular carcinoma in a subject, comprising administering a therapeutically effective amount of a peptide.

### Definitions

ALMS 1, Alström syndrome protein 1, is a protein encoded by the *ALMS1* gene. Mutations in the ALMS1 gene have been found to be causative for Alström syndrome. It is described in several databases, namely UniProt ID No Q8TCU4; Gene ID No 7840, HGNG ID No 428. Reference sequences are disclosed in Genbank under NM_015120.4 for mRNA and NP_055935.4 for protein.

The terms "Protein kinase C" and "PKC" (EC 2.7.11.13) are equivalent and refers to a family of protein kinase enzymes that are involved in controlling the function of other proteins through the phosphorylation of hydroxyl groups of serine and threonine amino acid residues on these proteins. PKC are typically activated by signals such as increases in the concentration of diacylglycerol (DAG) or calcium ions (Ca2+). PKC play important roles in several signal transduction cascades.

The PKC family comprises at least fifteen isozymes in humans, divided into three main subfamilies, conventional (or classical) PKCs, novel PKCs, and atypical PKCs.

Conventional (c)PKCs comprises the isoforms α, βI, βII, and γ. These PKCs require Ca²⁺, DAG, and a phospholipid such as phosphatidylserine for activation.

Novel (n)PKCs include the δ, ε, η, and θ isoforms. These PKCs require DAG, but do not require Ca2⁺ for activation.

Atypical (a)PKCs include the ζ, ι, and λ isoforms. These PKCs require neither Ca2+ nor diacylglycerol for activation.

Protein kinase C alpha type, also called αPKC, PKC-A or PKC-alpha, belongs to a family of serine- and threonine-specific protein kinases that can be activated by calcium and the second messenger diacylglycerol. It is described in several databases, namely UniProt ID No P17252, Gene ID No 9393, HGNG ID No 5578. Reference sequences are disclosed in Genbank under NM_02737.2 for mRNA and NP_002728.1 for protein. The protein sequence of human αPKC is disclosed in SEQ ID No 1.

"*consists of'*," "*consists essentially of*" *or* "*substantially comprises*": The description herein of any aspect or embodiment of the invention using terms such as reference to an element or elements is intended to provide support for a similar aspect or embodiment of the invention that "consists of," "consists essentially of" or "substantially comprises" that particular element or elements, unless otherwise stated or clearly contradicted by context. For instance, a peptide or protein described herein as comprising a particular sequence should be understood as also describing a peptide or protein consisting of that sequence, unless otherwise stated or clearly contradicted by context. By "consists essentially of" is intended that the peptide or protein consists of that sequence, but it may also include 1, 2, 3, 4, 5, 6, 7, 8, 9 or 10 substitutions, additions, deletions or a mixture thereof, preferably 1, 2, 3, 4, or 5 substitutions, additions, deletions or a mixture thereof. In particular, by "essentially consist in", it may be intended that the peptide may include 1, 2, 3, 4, 5, 6, 7, 8, 9 or 10 additional amino acids at the N and/or C-terminal end, preferably 1, 2, 3, 4, or 5 additional amino acids, and/or 1,2 or 3 substitutions, deletions , additions, or a mixture thereof.

As used herein, the term "substitution" refers to the exchange of a single amino-acid by another in a peptide sequence.

As used herein, the term "deletion" refers to the removal of a single amino-acid in a peptide sequence.

As used herein, the term "insertion" or "addition" are equivalent and refer to the addition of a single amino-acid in a peptide sequence.

In the peptide sequences disclosed herein, the amino acids are represented by their one letter code according to the following nomenclature: A: alanine; C: cysteine; D: aspartic acid; E: glutamic acid; F: phenylalanine; G: glycine; H: histidine; I: isoleucine; K: lysine; L: leucine ; M: methionine ; N: asparagine ; P: proline ; Q: glutamine ; R: arginine ; S: serine ; T: threonine ; V: valine ; W: tryptophane and Y: tyrosine.

As used herein, the terms "sequence identity" or "identity" refers to an exact amino acid to amino acid correspondence of two peptides. Percent of identity can be determined by a direct comparison of the sequence information between two molecules by aligning the sequences, counting the exact number of matches between the two aligned sequences, dividing by the length of the shorter sequence, and multiplying the result by 100.

The sequence identity can be determined by alignment of two peptide sequences using global or local alignment algorithms, depending on the length of the two sequences. Sequences of similar lengths are preferably aligned using a global alignment algorithms (e.g. Needleman Wunsch) which aligns the sequences optimally over the entire length, while sequences of substantially different lengths are preferably aligned using a local alignment algorithm (e.g. Smith Waterman). Sequences may then be referred to as "substantially identical" or "essentially similar" when they (when optimally aligned by for example the programs GAP or BESTFIT using default parameters) share at least a certain minimal percentage of sequence identity. GAP uses the Needleman and Wunsch global alignment algorithm to align two sequences over their entire length (full length), maximizing the number of matches and minimizing the number of gaps. A global alignment is suitably used to determine sequence identity when the two sequences have similar lengths.

By "increased", "increase" or "enhance" is intended to refer to a measurement increased by at least 10, 20, 30, 40, 50, 60, 70, 80 or 90 % when compared to the measurement measured in absence of the tested molecule in the same conditions. By "decreased" or "decrease" is intended to refer to a measurement decreased by at least 10, 20, 30, 40, 50, 60, 70, 80 or 90 % when compared to the measurement measured in absence of the tested molecule in the same conditions.

As used herein, the term "treatment", "treat" or "treating" refers to any act intended to ameliorate the health status of patients, such as cure, alleviate or delay of the disease. It includes preventive as well as therapeutic treatment. For instance, it may refer to a delay or a blockade of the evolution from NAFLD to NASH, from NASH to NASH with fibrosis, from NASH to cirrhosis, from NASH or cirrhosis to hepatocellular carcinoma. The term treatment designates in particular the correction, retardation, or reduction of the hepatic steatosis. The term "treatment" also designates an improvement in the liver steatosis, in liver inflammation, in liver fibrosis, in liver enzymes (aminotransferases such as AST and ALT), and/or in fatty liver index (Bedgni et al, BMC Gastroenterol. 2006 Nov 2;6:33). In particular, the treatment lowers or decreases or delays the in the liver steatosis, in liver inflammation, in liver fibrosis, in liver enzymes (aminotransferases such as AST and ALT), and/or in fatty liver index.

As used herein, the term "effective amount" refers to a quantity of a peptide of the present disclosure or of a pharmaceutical composition of the present disclosure which treats or delays the progression or onset of nonalcoholic fatty liver disease (NAFLD), non-alcoholic fatty liver (NAFL), non-alcoholic steatohepatitis (NASH), hepatic steatosis (fatty liver), liver fibrosis, liver inflammation, cirrhosis, or hepatocellular carcinoma.

As used herein, the terms "active principle", "active ingredient" and "active pharmaceutical ingredient" are equivalent and refers to a component of a pharmaceutical composition having a therapeutic effect.

As used herein, the term "therapeutic effect" refers to an effect induced by an active ingredient, such as a peptide of the present disclosure, or by a pharmaceutical composition according to the present disclosure, capable to treat or to delay the progression or onset of nonalcoholic fatty liver disease (NAFLD), non-alcoholic fatty liver (NAFL), non-alcoholic steatohepatitis (NASH), hepatic steatosis (fatty liver), liver fibrosis, liver inflammation, cirrhosis, or hepatocellular carcinoma.

As used herein, the term "excipient or pharmaceutically acceptable carrier" refers to any ingredient except active ingredients that is present in a pharmaceutical composition. Its addition may be aimed to confer a particular consistency or other physical or gustative properties to the final product. An excipient or pharmaceutically acceptable carrier must be devoid of any interaction, in particular chemical, with the actives ingredients.

As used herein, the terms "subject", "individual" or "patient" are interchangeable and refer to an animal, preferably to a mammal, even more preferably to a human, including adult, child, newborn and human at the prenatal stage.

In the present document, the term "about" refers to a range of values of ± 10% of the specified value. For example, "about 50" comprise values of ± 10% of 50, i.e. values in the range between 45 and 55. Preferably, the term "about" refers to a range of values of ± 5% of the specified value.

As used herein, "nonalcoholic fatty liver disease" and "NAFLD" refer to a disease defined by the presence of macro vascular steatosis in the presence of less than 20 gm of alcohol ingestion per day. NAFLD is the most common liver disease in the United States, and is commonly associated with insulin resistance/type 2 diabetes mellitus and obesity. NAFLD is manifested by steatosis, steatohepatitis, cirrhosis, and sometimes hepatocellular carcinoma. For a review of NAFLD, see Tolman and Dalpiaz (2007) Ther. Clin. Risk. Manag., 3(6): 1153-1163 the entire contents of which are incorporated herein by reference.

As used herein, the terms "steatosis," "hepatic steatosis," and "fatty liver" refer to the accumulation of triglycerides and other fats in the liver cells.

As used herein, the term "Nonalcoholic steatohepatitis" or "NASH" refers to liver inflammation and damage caused by a buildup of fat in the liver. NASH is part of a group of conditions called nonalcoholic fatty liver disease (NAFLD). NASH resembles alcoholic liver disease, but occurs in people who drink little or no alcohol. The major feature in NASH is fat in the liver, along with inflammation and damage. Most people with NASH feel well and are not aware that they have a liver problem. Nevertheless, NASH can be severe and can lead to cirrhosis, in which the liver is permanently damaged and scarred and no longer able to work properly. NASH is usually first suspected in a person who is found to have elevations in liver tests that are included in routine blood test panels, such as alanine aminotransferase (ALT) or aspartate aminotransferase (AST). When further evaluation shows no apparent reason for liver disease (such as medications, viral hepatitis, or excessive use of alcohol) and when x rays or imaging studies of the liver show fat, NASH is suspected. The only means of proving a diagnosis of NASH and separating it from simple fatty liver is a liver biopsy.

As used herein, the term "cirrhosis," defined histologically, is a diffuse hepatic process characterized by fibrosis and conversion of the normal liver architecture into structurally abnormal nodules.

NAFLD may be differentiated from NASH by the NAFLD Activity Score (NAS), the sum of the histopathology scores of a liver biopsy for steatosis (0 to 3), lobular inflammation (0 to 2), and hepatocellular ballooning (0 to 2). A NAS of <3 corresponds to NAFLD, 3-4 corresponds to borderline NASH, and >5 corresponds to NASH. The biopsy is also scored for fibrosis (0 to 4).

### Peptides

The peptide(s) according to the present disclosure is capable of decreasing the expression of FATP2 in adipose tissue.

The peptide(s) according to the present disclosure is capable of decreasing the phenomenon of steatosis in the liver.

The peptide according to the present disclosure comprises at least one methionine, one proline and one arginine.

In one aspect, the peptide of the present disclosure has an alpha helix structure. As used herein, the terms "alpha helix" "α-helix", "classic Pauling-Corey-Branson α-helix" and "3.613-helix" are equivalent and refer to each other. The term "alpha helix" refers to a common motif in the secondary structure of proteins which is a right hand-coiled or spiral conformation (helix) in which every backbone N-H group donates a hydrogen bond to the backbone C=O group of the amino acid located three or four residues earlier along the protein sequence. An alpha helix has an average number of residues per helical turn of about 3.6 residues and 13 atoms are involved in the ring formed by the hydrogen bond.

In a particular embodiment, the peptide of the present disclosure has an alpha helix structure and/or has a sequence which is predictive of an alpha helix structure. Methods to determine the structure of a peptide are well known from the man skilled in the art, such as Circular Dichroism or NMR. Likewise, methods to predict an alpha helix structure of a peptide are well known from the man skilled in the art such as STRIDE (Frishman D., Argos P., Proteins, vol. 23, no 4, 1995, p. 566-579) ; DEFINE (Richards F. M., Kundrot C. E., Proteins, vol. 3, no 2, 1988, p. 71-84); DSSP (Touw et al. Nucleic Acids Research 2015; 43: D364-D368 ; Kabsch & Sander. Biopolymers. 1983, 22, 2577-2637).

In another particular embodiment, the peptide according to the present disclosure is designed or modified in order to maintain it in an alpha helical conformation. As known in the art, this can be achieved via a variety of methods, including modification of the amino acid sequence with substitution of amino acids not critical for biological effects, use of non-natural amino acids, peptide cyclization, and modifications to the peptide backbone or addition of chemical links between amino acids in the peptide chain. Such modifications can be made to peptides, for example, to increase their thermal and protease stability.

In particular, the peptide of the present disclosure is modified by a chemical cross-link. For instance, the peptide can be a stapled peptide. In one embodiment, the peptide of the present disclosure is stapled. The term "stapled peptide" or "stitched peptide", as used herein, refers to an artificially modified peptide in which the peptide secondary structure is stabilized with one or more artificial molecular crosslinks (bridges) that connect adjacent turns of α-helices in the peptide. The methods for preparing stapled peptides are well known in the art, for instance in Verdine & Hilinski (2012, Methods Enzymol, 503, 3-33), WO10033617 and WO10011313, the disclosure of which is incorporated herein by reference.

In one embodiment, the crosslinks of the stapled peptide of the present disclosure are i+3, and/or i+4, and/or i+7 crosslinks. In a peptide, a "i+3 crosslink" is a crosslink between an amino acid, the "i" amino acid, and another amino acid present at a distance of 3 amino acid residues from the i amino acid. In a peptide, a "i+4 crosslink" is a crosslink between an amino acid, the "i" amino acid, and another amino acid present at a distance of 4 amino acid residues from the i amino acid. In a peptide, a "i+7 crosslink" is a crosslink between an amino acid, the "i" amino acid, and another amino acid present at a distance of 7 amino acid residues from the i amino acid.

In a particular embodiment, the peptide according to the present disclosure is a cyclic peptide. As used herein, the term "cyclic peptide" or "circular peptide" are equivalent and refers to a peptide in which the N-terminus and the C-terminus, or the N-terminus and the side chain of another amino acid, preferably the C-terminal amino acid, or the C-terminus and the side chain of another amino acid, preferably the N-terminal amino acid, or the side chain of an amino acid and the side chain of another amino acid, preferably the N-terminal amino acid and the C-terminal amino acid, are linked with a covalent bond that generates a ring structure. As used herein, the term "N-terminus", "amino-terminus", "NH2-terminus", "N-terminal end" and "amine-terminus" are equivalent and refer to the free amine group (-NH2) present on the first amino acid of the peptide. As used herein, the term "C-terminus", "carboxyl-terminus", "carboxy-terminus", "C-terminal end", and "COOH-terminus" are equivalent and refer to the free carboxyl group (-COOH) present on the last amino acid of the peptide.

The peptide may comprise a sequence according to one of the following formulae: M-(X)m-P-(X)n-R or M-(X)m-R-(X)n-P or P-(X)m-M-(X)n-R or P-(X)m-R-(X)n-M or R-(X)m-P-(X)n-M or R-(X)m-M-(X)n-P, preferably M-(X)m-R-(X)n-P or P-(X)m-R-(X)n-M or R-(X)m-M-(X)n-P or M-(X)m-P-(X)n-R, with X is any amino acid, m and n are integers from 1 to 30, and m + n is less than 50.

In these sequences, m and n are integers comprised between 1 and 30, preferably between 1 and 20, more preferably between 2 and 15, still preferably between 2 and 10, even more preferably between 2 and 7. Alternatively, m and n can be integers comprised between 3 and 5.

In these sequences, m + n is less than 50, preferably less than 30, more preferably less than 20. Alternatively, m + n can be less than 15.

In one embodiment, m and n are integers from 1 to 20 and m + n is less than 30.

In these sequences, X can be any amino acid, including M, R or P. Alternatively, X can be any amino acid except M, R and P.

In particular, X can be an amino acid favorable to an α-helix secondary structure. For instance, such an amino acid can be selected from the group consisting of A, R, D, N, C, G, Q, E, H, L, K, M, F, S, W and Y, preferably from the group consisting of A, D, N, C, G, Q, E, H, L, K, F, S, W and Y.

The peptide according to the present disclosure has a length of less than 120 amino acids. In one embodiment, the peptide according to the present disclosure has a length of less than 80 amino acids, more preferably less than 60 amino acids, still preferably less than 40 amino acids, and even more preferably less than 30 amino acids. In a particular embodiment, the peptide according to the present disclosure has a length of less than 25 amino acids. In another particular embodiment, the peptide according to the present disclosure has a length of less than 20 amino acids, preferably of less than 15 amino acids. Preferably, the peptide has a minimum length greater than 3, 4, 5, 6, 7, 8, 9, 10, 11, 12, 13, 14, 15, 16, 17, 18, 19, or 20 amino acids. For instance, the peptide has a length of at least 8 amino acids and less than 40 amino acids, preferably a length of at least 10 amino acids and less than 30 amino acids; more preferably of at least 12 amino acids and less than 25 amino acids.

In one embodiment, the peptide according to the present disclosure is capable of decreasing the expression of FATP2 in adipose tissue.

FATP2 is also called Solute Carrier Family 27 Member 2 (SLC27A2). This protein is disclosed in the database UniProtKB under 014975. The gene is described in UniGene database under Hs.11729. Sequences of reference can be found in NCBI under NP_003636.2 and NM_003645.3 for the isoform 1 and under NP_001153101.1 and NM_001159629.1. for the isoform 2.

By "decreased" or "decrease" is intended to refer to an expression decreased by at least 10, 20, 30, 40, 50, 60, 70, 80 or 90 % when compared to the expression measured in absence of the peptide in the same conditions. The expression can be measured either at the protein level (e.g., with antibodies) or at the mRNA level.

The expression can be measured at the protein level by any available method such as immuno-histochemistry, semi-quantitative Western-blot or by protein or antibody arrays. Antibodies directed to FATP2 are commercially available, for instance from Origene, ref TA350424 or TA333990; or Santa Cruz Biotechnology, ref sc-393906.

The expression can also be measured at the mRNA level by any available method. Preferably, the expression level of FATP2 is determined by measuring the quantity of the mRNA transcripts by quantitative RT-PCR, real time quantitative RT-PCR, Nanostring technology PCR or by high-throughput sequencing technology such as RNA-Seq or sequencing technologies using microfluidic systems. More specifically, the expression is measured by the method specified in the Example section.

The effect on the FATP2 expression caused by the peptide in the adipose tissue is preferably specific to FATP2. In particular, the peptide has no or less effect on the expression of the other FATPs, i.e. FATP1, FATP3, FATP4, FATP5 and FATP6, in the adipose tissue.

In one embodiment, the peptide according to the present disclosure is capable of decreases the liver steatosis, the amount of fat in liver, the size of fat droplets in liver, and/or the fatty liver index.

In a particular embodiment, the peptide according to the present disclosure presents two, three, four or all following features:
- it modifies the expression levels of the FATPs expression in adipose tissue, preferentially it decreases the FATP2 expression in adipose tissue;
- it decreases the liver steatosis, the amount of fat in liver, the size of fat droplets in liver, and/or the fatty liver index;
- it comprises at least one methionine, one proline and one arginine;
- it has a length of at least 8 amino acids and less than 40 amino acids, preferably a length of at least 10 amino acids and less than 30 amino acids, more preferably of at least 12 amino acids and less than 25 amino acids;
- it adopts a secondary structure which is a helix, preferably an alpha helix;
- it is modified by a cross-link.

In a more specific embodiment, the peptide according to the present disclosure presents the following features:
- it decreases the liver steatosis, the amount of fat in liver, the size of fat droplets in liver, and/or the fatty liver index;
- it comprises at least one methionine, one proline and one arginine;
- it has a length of at least 8 amino acids and less than 40 amino acids, preferably a length of at least 10 amino acids and less than 30 amino acids, more preferably of at least 12 amino acids and less than 25 amino acids;
- it adopts a secondary structure which is a helix, preferably an alpha helix.

In another more specific embodiment, the peptide according to the present disclosure presents the following features:
- it decreases the FATP2 expression in adipose tissue;
- it comprises at least one methionine, one proline and one arginine;
- it has a length of at least 8 amino acids and less than 40 amino acids, preferably a length of at least 10 amino acids and less than 30 amino acids, more preferably of at least 12 amino acids and less than 25 amino acids;
- it adopts a secondary structure which is a helix, preferably an alpha helix.

In another particular embodiment, the peptide sequence according to the present disclosure comprises, consists essentially in or consists in the sequence of a segment of a PKC (Protein Kinase C). In one embodiment, the PKC is selected from conventional PKC, novel PKC and atypical PKC. In a particular embodiment, the PKC is selected from conventional PKC. Still preferably, the PKC is selected from the group consisting of α, βI, βII, and γ PKCs. Yet preferably, the PKC is selected from the group consisting of α, βI, and βII PKCs. In a more specific embodiment, the PKC is an α PKC, preferably a human α PKC, more preferably a human α PKC of SEQ ID No 1.

In one embodiment, said sequence of a segment of a PKC belongs to the sequence between positions 336 and 672 of SEQ ID No 1. For instance, the sequence of a segment of a PKC may belong to the sequences between positions 336 and 432 of SEQ ID No 1, between positions 434 and 544 of SEQ ID No 1, or between positions 568 and 596 of SEQ ID No 1.

In one embodiment, the sequence of a segment of a PKC does not include the following residues: F114, D116, C118, L121, N138, Q142, I145, P148, G433, E545, S562, S566, F597, D601, W602, K604, E606, G620, T631, V664, and I667 of SEQ ID No 1, preferably G433, E545, S562, S567, F597, D601, W602, K604, E606, G620, T631, V664, and I667 of SEQ ID No 1.

Preferably, said sequence of a segment of a PKC corresponds to at least 10%, 20%, 30%, 40%, 50%, 60%, 70%, 80%, 90%, 95%, 99% of the sequence of the peptide. In a particular embodiment, the peptide sequence according to the present disclosure consist in the sequence of a segment of SEQ ID No 1.

Preferably, said at least one methionine and/or one proline and/or one arginine are included in the sequence of a segment of a PKC. More preferably, said at least one methionine and one proline and one arginine are comprised in the sequence of a segment of a PKC.

The peptide according to the present disclosure may comprise substitutions, deletions and/or additions.

Preferably, the peptide comprises no more than 20, preferably no more than 15, more preferably no more than 10, substitutions, deletions, additions, or a mixture thereof. In a particularly preferred embodiment, the peptide may comprise 1, 2, 3, 4, 5, 6, 7, 8, 9 or 10 substitution(s), deletion(s), addition(s), or a mixture thereof, preferably 1, 2, 3, 4, or 5, more preferably 1, 2 or 3. The substitution(s), deletion(s), addition(s) are not introduced at the positions of said at least one methionine, one proline, and one arginine.

In an alternative embodiment, the peptide has at least 70%, 75%, 80%, 85%, 90%, 95%, 99% of identity with the sequence of a segment of a PKC, preferably of SEQ ID No 1. In one embodiment, the part of the sequence of the peptide corresponding to SEQ ID No 1 has at least 70%, 75%, 80%, 85%, 90%, 95%, of identity with the sequence of a segment of SEQ ID No 1.

In another particular embodiment, the sequence of the peptide according to the present disclosure may comprise, consist essentially in or consist in a sequence selected from the group consisting of:
- MVEKRVLALLDKP (SEQ ID No 2);
- PFLTQLHSCFQTVDRLYFVM (SEQ ID No 3);
- RLYFVMEYVNGGDLMYHIQQVGKFKEP (SEQ ID No 4);
- MYHIQQVGKFKEPQAVFYAAEISIGLFFLHKR (SEQ ID No 5);
- PQAVFYAAEISIGLFFLHKRGIIYRDLKLDNVM (SEQ ID No 6);
- MDGVTTRTFCGTP (SEQ ID No 7);
- MTKHPAKR (SEQ ID No 8); and
- RKGTEELYAIKILKKDVVIQDDDVECTMVEKRVLALLDKP (SEQ ID No 12).
- RVLALLDKPPFLTQLHSCFQTVDRLYFVM (SEQ ID No 13); and
- RTFCGTPDYIAPEIIAYQPYGKSVDWWAYGVLLYEM (SEQ ID No 14)
and optionally it comprises 1, 2, 3, 4, 5, 6, 7, 8, 9, or 10 substitution(s), deletion(s), addition(s), or a mixture thereof. Preferably, it may comprise 1, 2, 3, 4, or 5 substitution(s), deletion(s), addition(s), or a mixture thereof. More preferably, it may comprise 1, 2, or 3 substitution(s), deletion(s), addition(s), or a mixture thereof. Preferably, the substitution(s), deletion(s), addition(s) are such that the at least one M, P and R are conserved. More preferably, the substitution(s), deletion(s), addition(s) are such that M, P and R are conserved. In addition, the number of substitution(s), deletion(s), addition(s) is chosen in order to keep at least 50%, 60% 70%, 75%, 80%, 85%, 90%, 95% of identity with the sequence of a segment of SEQ ID No 1.

In one aspect, the sequence of the peptide according to the present disclosure comprises, consists essentially in or consists in a sequence selected from the group consisting of:
- MVEKRVLALLDKP (SEQ ID No 2);
- PFLTQLHSCFQTVDRLYFVM (SEQ ID No 3);
- RLYFVMEYVNGGDLMYHIQQVGKFKEP (SEQ ID No 4);
- MYHIQQVGKFKEPQAVFYAAEISIGLFFLHKR (SEQ ID No 5);
- PQAVFYAAEISIGLFFLHKRGIIYRDLKLDNVM (SEQ ID No 6);
- MDGVTTRTFCGTP (SEQ ID No 7); and
- MTKHPAKR (SEQ ID No 8);
and optionally it comprises 1, 2, 3, 4, 5, 6, 7, 8, 9, or 10 substitution(s), deletion(s), addition(s), or a mixture thereof. Preferably, it may comprise 1, 2, 3, 4, or 5 substitution(s), deletion(s), addition(s), or a mixture thereof. More preferably, it may comprise 1, 2, or 3 substitution(s), deletion(s), addition(s), or a mixture thereof. Preferably, the substitution(s), deletion(s), addition(s) are such that the at least one M, P and R are conserved. More preferably, the substitution(s), deletion(s), addition(s) are such that M, P and R are conserved. In addition, the number of substitution(s), deletion(s), addition(s) is chosen in order to keep at least 50%, 60% 70%, 75%, 80%, 85%, 90%, 95% of identity with the sequence of a segment of SEQ ID No 1.

In another aspect, the sequence of the peptide according to the present disclosure comprises, consists essentially in or consists in a sequence selected from the group consisting of:
- MVEKRVLALLDKP (SEQ ID No 2);
- PFLTQLHSCFQTVDRLYFVM (SEQ ID No 3);
- PQAVFYAAEISIGLFFLHKRGIIYRDLKLDNVM (SEQ ID No 6)
- MDGVTTRTFCGTP (SEQ ID No 7);
- MTKHPAKR (SEQ ID No 8);
and optionally it comprises 1, 2, 3, 4, 5, 6, 7, 8, 9, or 10 substitution(s), deletion(s), addition(s), or a mixture thereof. Preferably, it may comprise 1, 2, 3, 4, or 5 substitution(s), deletion(s), addition(s), or a mixture thereof. More preferably, it may comprise 1, 2, or 3 substitution(s), deletion(s), addition(s), or a mixture thereof. Preferably, the substitution(s), deletion(s), addition(s) are such that the at least one M, P and R are conserved. More preferably, the substitution(s), deletion(s), addition(s) are such that M, P and R are conserved. In addition, the number of substitution(s), deletion(s), addition(s) is chosen in order to keep at least 50%, 60% 70%, 75%, 80%, 85%, 90%, 95% of identity with the sequence of a segment of SEQ ID No 1.

In a particular aspect, the sequence of the peptide according to the present disclosure comprises, consists essentially in or consists in a sequence selected from the group consisting of:
- MVEKRVLALLDKP (SEQ ID No 2);
- MDGVTTRTFCGTP (SEQ ID No 7);
- MTKHPAKR (SEQ ID No 8);
and optionally it comprises 1, 2, 3, 4, 5, 6, 7, 8, 9, or 10 substitution(s), deletion(s), addition(s), or a mixture thereof. Preferably, it may comprise 1, 2, 3, 4, or 5 substitution(s), deletion(s), addition(s), or a mixture thereof. More preferably, it may comprise 1, 2, or 3 substitution(s), deletion(s), addition(s), or a mixture thereof. Preferably, the substitution(s), deletion(s), addition(s) are such that the at least one M, P and R are conserved. More preferably, the substitution(s), deletion(s), addition(s) are such that M, P and R are conserved. In addition, the number of substitution(s), deletion(s), addition(s) is chosen in order to keep at least 50%, 60% 70%, 75%, 80%, 85%, 90%, 95% of identity with the sequence of a segment of SEQ ID No 1.

Alternatively, the sequence of the peptide according to the present disclosure comprises, consists essentially in or consists in a sequence selected from the group consisting of:
- VECTMVEKRVLALLDKPPFLTQLHS (SEQ ID No 9);
- MCKEHMMDGVTTRTFCGTPD (SEQ ID No 10); and
- SICKGLMTKHPAKRLGCGPEG (SEQ ID No 11);
and optionally it comprises 1, 2, 3, 4, 5, 6, 7, 8, 9, 10, 11, 12, 13, 14, or 15 substitution(s), deletion(s), addition(s), or a mixture thereof. Preferably, it may comprise 1, 2, 3, 4, 5, 6, 7, 8, 9, or 10 substitution(s), deletion(s), addition(s), or a mixture thereof. More preferably, it may comprise 1, 2, 3, 4, or 5 substitution(s), deletion(s), addition(s), or a mixture thereof. Preferably, the substitution(s), deletion(s), addition(s) are such that the at least one M, P and R are conserved. More preferably, the substitution(s), deletion(s), addition(s) are such that M, P and R are conserved. In addition, the number of substitution(s), deletion(s), addition(s) is chosen in order to keep at least 50%, 60% 70%, 75%, 80%, 85%, 90%, 95% of identity with the sequence of a segment of SEQ ID No 1.

In a particular aspect, the peptide comprises, consists essentially in or consists in a sequence selected from the group consisting of:
- VECTM**V**EKRVLA**L**LDKPPFLTQLHS (SEQ ID No 9);
- MC**K**EHMMDG**V**TTRTFCGTPD (SEQ ID No 10); and
- S**I**CKGLMT**K**HPAKRLGCGPEG (SEQ ID No 11);
wherein the residues which are bold and underlined carry the stapling.

Optionally the peptide according to the present disclosure further comprises 1, 2, 3, 4, 5, 6, 7, 8, 9 or 10 substitution(s), deletion(s), addition(s), or a mixture thereof in the above listed sequences. Preferably, it may comprise 1, 2, 3, 4, 5, 6, 7, 8, 9, or 10 substitution(s), deletion(s), addition(s), or a mixture thereof. More preferably, it may comprise 1, 2, 3, 4, or 5 substitution(s), deletion(s), addition(s), or a mixture thereof. The substitution(s), deletion(s), addition(s) is chosen in order to conserve the at least one M, P and R and the two residues used for the stapling. More preferably, the substitution(s), deletion(s), addition(s) are such that M, P and R are conserved.

The peptide according to the present disclosure may further comprise a moiety facilitating its cellular uptake or entry, in particular a PTD (protein transduction domain). PTD generally comprises a certain amino acid sequence of 10 to 20 amino acids (Matsushita and Matsui, (2005), J Mol Med 83, 324-328; Vivès et al, Biochimic et Biophysica Acta, 2008, 1786, 126-138). PTD is mainly composed of basic amino acids such as arginine or lysine, and representative examples of the PTD include arginine rich peptides such as poly R₈ (RRRRRRRR (SEQ ID No 15)) or (RRPRRPRRPRRPRRP (SEQ ID No 16)), antennapedia or penetratin peptide such as (RQIKIWFQNRRMKWKK (SEQ ID No 17)) or HIV-Tat (YGRKKRRQRRR (SEQ ID No 18)).

The peptide according to the present disclosure can be made of natural amino acids and/or unnatural amino acids, preferably at least one methionine, one proline, and one arginine are natural amino acids. The term "unnatural amino acids" is defined as an analog or derivative of a natural amino acid (i.e., Alanine, Valine, Glycine, Leucine, Isoleucine, Lysine, Arginine, Glutamic acid, Glutamine, Aspartic acid, Asparagine, Histidine, Tyrosine, Phenylalanine, Tryptophan, Serine, Proline, Threonine, Cysteine, Methionine). They present a modified side chain, e.g. shorter, longer or with different functional groups. Isomers D and L are contemplated, in particular because isomers D are not sensible to proteases. In addition, modifications in some or all peptide bounds are also contemplated in order to increase the proteolysis resistance, in particular by (-CO-NH-) by (-CH₂-NH-), (-NH-CO-), (-CH₂-O-), (-CH₂-S-), (-CH₂-CH₂-), (-CO-CH₂-), (-CHOH-CH₂-), (-N=N-), and/or (-CH=CH-). The peptide can present a carboxylic C terminal end (-COO⁻) and an amide one (-CONH₂). The peptide can also be D-retro-inverso sequence of a peptide as disclosed herein. The N terminal can be modified, especially with an acetyl radical.

Optionally, the peptide can be PEGylated in order to increase its stability. Further optionally the peptide can be formulated in non-aqueous protic solvent solutions such as propylene glycol and polyethylene glycol. The peptide may also be packaged into poly lactic co-glycolic acid microsphere depot formulation. Many sustained-release delivery systems exist, and many of these are appropriate for use in the present disclosure. For example, polymer-based slow-release compositions based upon degradable polymers such as PLGA, poly-lactate or poly-glycolate are suitable, as are lipid-based depot compositions, such as those described in WO2005/117830 and/or WO2006/075124, the complete disclosures of which are being hereby incorporated by reference. The formulation of active agents into biodegradable polymer depot formulations is well established and well known in the art, and the peptides of the present disclosure may thus be formulated with these using known methods. Preferably, the composition of the present disclosure is capable of releasing the peptide at a functional concentration for at least 1 month.

In an additional aspect, the peptide according to the present disclosure decreases the phenomenon of steatosis in the liver. The phenomenon of steatosis in the liver can be assessed by any method known from the man skilled in the art. In particular, it is assessed by the method described in the example section. For instance, the steatosis can be measured by imaging or biopsy. Peptides that decrease the phenomenon of steatosis in the liver can be conveniently screened for using any technology known in the art. In particular, a method for assessing the steatosis in the liver can comprise any method suitable for measuring the fat in the liver, the size of the lipid droplets in the liver and/or measuring the fatty liver index (Bedgni et al, BMC Gastroenterol. 2006 Nov 2;6:33).

By "a peptide" is intended to refer to a peptide as disclosed above or a combination of different peptides as disclosed above. For instance, 2, 3, 4, 5 or 6 different peptides can be used, preferably 2 or 3, more preferably 2.

In one embodiment, the combination of peptides comprises 2 or 3, preferably 2, peptides selected from the group consisting of:
- MVEKRVLALLDKP (SEQ ID No 2);
- PFLTQLHSCFQTVDRLYFVM (SEQ ID No 3);
- RLYFVMEYVNGGDLMYHIQQVGKFKEP (SEQ ID No 4);
- MYHIQQVGKFKEPQAVFYAAEISIGLFFLHKR (SEQ ID No 5);
- PQAVFYAAEISIGLFFLHKRGIIYRDLKLDNVM (SEQ ID No 6);
- MDGVTTRTFCGTP (SEQ ID No 7);
- MTKHPAKR (SEQ ID No 8); and
- RKGTEELYAIKILKKDVVIQDDDVECTMVEKRVLALLDKP (SEQ ID No 12).
- RVLALLDKPPFLTQLHSCFQTVDRLYFVM (SEQ ID No 13); and
- RTFCGTPDYIAPEIIAYQPYGKSVDWWAYGVLLYEM (SEQ ID No 14)
and optionally it comprises 1, 2, 3, 4, 5, 6, 7, 8, 9, or 10 substitution(s), deletion(s), addition(s), or a mixture thereof.

In one embodiment, the combination of peptides comprises 2 or 3, preferably 2, peptides selected from the group consisting of:
- MVEKRVLALLDKP (SEQ ID No 2);
- PFLTQLHSCFQTVDRLYFVM (SEQ ID No 3);
- RLYFVMEYVNGGDLMYHIQQVGKFKEP (SEQ ID No 4);
- MYHIQQVGKFKEPQAVFYAAEISIGLFFLHKR (SEQ ID No 5);
- PQAVFYAAEISIGLFFLHKRGIIYRDLKLDNVM (SEQ ID No 6);
- MDGVTTRTFCGTP (SEQ ID No 7); and
- MTKHPAKR (SEQ ID No 8);
and optionally it comprises 1, 2, 3, 4, 5, 6, 7, 8, 9, or 10 substitution(s), deletion(s), addition(s), or a mixture thereof.

In one embodiment, the combination of peptides comprises 2 or 3, preferably 2, peptides selected from the group consisting of:
- MVEKRVLALLDKP (SEQ ID No 2);
- PFLTQLHSCFQTVDRLYFVM (SEQ ID No 3);
- PQAVFYAAEISIGLFFLHKRGIIYRDLKLDNVM (SEQ ID No 6)
- MDGVTTRTFCGTP (SEQ ID No 7);
- MTKHPAKR (SEQ ID No 8);
and optionally it comprises 1, 2, 3, 4, or 5 substitution(s), deletion(s), addition(s), or a mixture thereof.

In one embodiment, the combination of peptides comprises 2 or 3, preferably 2, peptides selected from the group consisting of:
- MVEKRVLALLDKP (SEQ ID No 2);
- MDGVTTRTFCGTP (SEQ ID No 7);
- MTKHPAKR (SEQ ID No 8);
and optionally it comprises 1, 2, 3, 4, or 5 substitution(s), deletion(s), addition(s), or a mixture thereof.

In one embodiment, the combination of peptides comprises 2 or 3, preferably 2, peptides selected from the group consisting of:
- VECTMVEKRVLALLDKPPFLTQLHS (SEQ ID No 9);
- MCKEHMMDGVTTRTFCGTPD (SEQ ID No 10); and
- SICKGLMTKHPAKRLGCGPEG (SEQ ID No 11);
and optionally it comprises 1, 2, 3, 4, 5, 6, 7, 8, 9, 10, 11, 12, 13, 14, or 15 substitution(s), deletion(s), addition(s), or a mixture thereof, preferably 1, 2, 3, 4, 5, 6, 7, 8, 9, or 10 substitution(s), deletion(s), addition(s), or a mixture thereof, more preferably, 1, 2, 3, 4, or 5 substitution(s), deletion(s), addition(s), or a mixture thereof.

In one particular embodiment, the combination of peptides comprises the peptides:
- VECTMVEKRVLALLDKPPFLTQLHS (SEQ ID No 9); and
- SICKGLMTKHPAKRLGCGPEG (SEQ ID No 11);
and optionally it comprises 1, 2, 3, 4, 5, 6, 7, 8, 9, 10, 11, 12, 13, 14, or 15 substitution(s), deletion(s), addition(s), or a mixture thereof, preferably 1, 2, 3, 4, 5, 6, 7, 8, 9, or 10 substitution(s), deletion(s), addition(s), or a mixture thereof, more preferably, 1, 2, 3, 4, or 5 substitution(s), deletion(s), addition(s), or a mixture thereof.

In one embodiment, the combination of peptides comprises 2 or 3, preferably 2, peptides selected from the group consisting of:
selected from the group consisting of:
   - VECTM**V**EKRVLA**L**LDKPPFLTQLHS (SEQ ID No 9);
   - MC**K**EHMMDG**V**TTRTFCGTPD (SEQ ID No 10); and
   - S**I**CKGLMT**K**HPAKRLGCGPEG (SEQ ID No 11);
   wherein the residues which are bold and underlined carry the stapling.

In one particular embodiment, the combination of peptides comprises the peptides:
- VECTM**V**EKRVLA**L**LDKPPFLTQLHS (SEQ ID No 9); and
- S**I**CKGLMT**K**HPAKRLGCGPEG (SEQ ID No 11);
wherein the residues which are bold and underlined carry the stapling.

### Combinations

The peptide(s) according to the present disclosure can be used in combination with one or more additional active drugs, for instance an anti-diabetic drug, a hypolipidemic agent, an anti-obesity agent, an anti-hypertensive agent, an anti-steatotic drug and an agonist of peroxisome proliferator-activator receptors. Accordingly, the present invention relates to:
- a peptide as disclosed herein for use in the treatment or prevention of a disease selected from the group consisting of nonalcoholic fatty liver disease (NAFLD), non-alcoholic fatty liver (NAFL), non-alcoholic steatohepatitis (NASH), hepatic steatosis (fatty liver), liver fibrosis, liver inflammation, cirrhosis, or hepatocellular carcinoma in combination with one or more additional active drugs, in particular as disclosed herein;
- a pharmaceutical composition comprising a peptide as disclosed herein and one or more additional active drugs, in particular as disclosed herein, for use in the treatment or prevention of a disease selected from the group consisting of nonalcoholic fatty liver disease (NAFLD), non-alcoholic fatty liver (NAFL), non-alcoholic steatohepatitis (NASH), hepatic steatosis (fatty liver), liver fibrosis, liver inflammation, cirrhosis, or hepatocellular carcinoma;
- a product, combined preparation or kit comprising a peptide according to the present disclosure and one or more additional active drugs, in particular as disclosed herein, for simultaneous, separate or sequential use in the treatment or prevention of a disease selected from the group consisting of nonalcoholic fatty liver disease (NAFLD), non-alcoholic fatty liver (NAFL), non-alcoholic steatohepatitis (NASH), hepatic steatosis (fatty liver), liver fibrosis, liver inflammation, cirrhosis, or hepatocellular carcinoma;
- the use of a peptide for the manufacture of a medicine for the treatment or prevention of a disease selected from the group consisting of nonalcoholic fatty liver disease (NAFLD), non-alcoholic fatty liver (NAFL), non-alcoholic steatohepatitis (NASH), hepatic steatosis (fatty liver), liver fibrosis, liver inflammation, cirrhosis, or hepatocellular carcinoma in combination with one or more additional active drugs, in particular as disclosed herein;
- the use of a peptide as disclosed herein and one or more additional active drugs, in particular as disclosed herein, for the manufacture of a medicine for the treatment or prevention of a disease selected from the group consisting of nonalcoholic fatty liver disease (NAFLD), non-alcoholic fatty liver (NAFL), non-alcoholic steatohepatitis (NASH), hepatic steatosis (fatty liver), liver fibrosis, liver inflammation, cirrhosis, or hepatocellular carcinoma in combination with one or more additional active drugs, in particular as disclosed herein;
- a method for the treatment or prevention of a disease selected from the group consisting of nonalcoholic fatty liver disease (NAFLD), non-alcoholic fatty liver (NAFL), non-alcoholic steatohepatitis (NASH), hepatic steatosis (fatty liver), liver fibrosis, liver inflammation, cirrhosis, or hepatocellular carcinoma in a subject, comprising administering a therapeutically effective amount of a peptide as disclosed herein and a therapeutically effective amount of one or more additional active drugs, in particular as disclosed herein;
- a method for the treatment or prevention of a disease selected from the group consisting of nonalcoholic fatty liver disease (NAFLD), non-alcoholic fatty liver (NAFL), non-alcoholic steatohepatitis (NASH), hepatic steatosis (fatty liver), liver fibrosis, liver inflammation, cirrhosis, or hepatocellular carcinoma in a subject, comprising administering a therapeutically effective amount of a pharmaceutical composition comprising a peptide as disclosed herein and one or more additional active drugs, in particular as disclosed herein..

In particular, a therapeutic or sub-therapeutic effective amount of one or more additional active drugs can be used. By "sub-therapeutic" is intended to refer to an amount that can be for instance 90, 80, 70, 60, 50, 40, 30, 20 or 10 % of the conventional therapeutic dosage (in particular for the same indication and the same administration route).

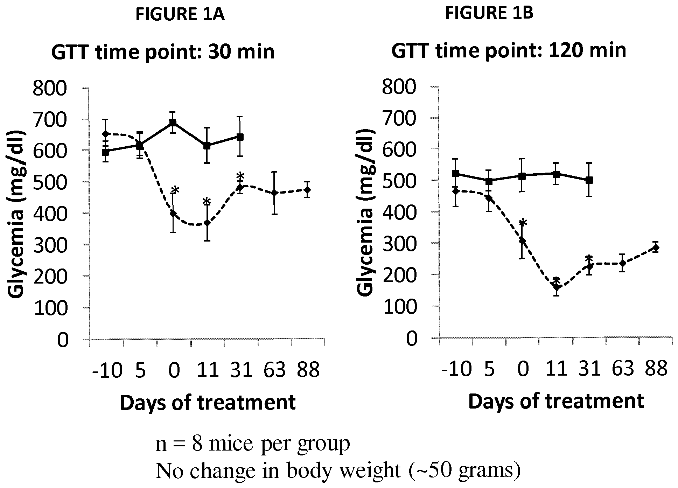

The anti-diabetic drug can be for instance insulin, insulin derivatives and mimetics; insulin secretagogues such as the sulfonylureas (e.g., chlorpropamide, tolazamide, acetohexamide, tolbutamide, glyburide, glimepiride, glipizide); gliflozins such as canagliflozin, emplagliflozin and dapagliflozin; glyburide and Amaryl; liraglutide (NN2211); insulinotropic sulfonylurea receptor ligands such as meglitinides, e.g. nateglinide and repaglinide; thiazolidinediones (e.g., rosiglitazone (AVANDIA), troglitazone (REZULIN), pioglitazone (ACTOS), balaglitazone, rivoglitazone, netoglitazone, troglitazone, englitazone, ciglitazone, adaglitazone, darglitazone that enhance insulin action (e.g., by insulin sensitization), thus promoting glucose utilization in peripheral tissues; protein tyrosine phosphatase- IB (PTP-1B) inhibitors such as PTP-112; Cholesteryl ester transfer protein (CETP) inhibitors such as torcetrapib, GSK3 (glycogen synthase kinase-3) inhibitors such as SB-517955, SB-4195052, SB- 216763, NN-57-05441 and NN-57-05445; RXR ligands such as GW-0791 and AGN-194204; sodium-dependent glucose cotransporter inhibitors such as T-1095; glycogen phosphorylase A inhibitors such as BAY R3401; biguanides such as metformin and other agents that act by promoting glucose utilization, reducing hepatic glucose production and/or diminishing intestinal glucose output; alpha-glucosidase inhibitors such as acarbose and migiitoi) and other agents that slow down carbohydrate digestion and consequently absorption from the gut and reduce postprandial hyperglycemia; GLP-1 (glucagon like peptide- 1), GLP-1 analogs such as Exendin-4 and GLP-1 mimetics; and DPPIV (dipeptidyl peptidase IV) inhibitors such as vildagliptin. It can also be an anti-diabetic drug described in Expert Opin Investig Drugs 2003, 12(4): 623-633, figures 1 to 7.

The hypolipidemic agent can be for instance 3-hydroxy-3-methyl-glutaryl coenzyme A (HMG- CoA) reductase inhibitors, e.g. lovastatin, pitavastatin, simvastatin, pravastatin, cerivastatin, mevastatin, velostatin, fluvastatin, dalvastatin, atorvastatin, rosuvastatin and rivastatin; squalene synthase inhibitors; FXR (farnesoid X receptor) and LXR (liver X receptor) ligands such as obeticholic acid; bile acid sequenstrants, such as cholestyramine and colesevelam; fibrates; nicotinic acid and aspirin; aramchol, a transmembrane G protein-coupled receptor (TGR) 5 agonist.

The anti-obesity agent can be for instance orlistat, rimonabant, phentermine, topiramate, qnexa, and locaserin.

The anti-hypertensive agent can be for instance loop diuretics such as ethacrynic acid, furosemide and torsemide; angiotensin converting enzyme (ACE) inhibitors such as benazepril, captopril, enalapril, fosinopril, lisinopril, moexipril, perinodopril, quinapril, ramipril and trandolapril; inhibitors of the Na-K-ATPase membrane pump such as digoxin; neutralendopeptidase (NEP) inhibitors such as sacubitril; ACE/NEP inhibitors such as omapatrilat, sampatrilat and fasidotril; angiotensin II antagonists such as candesartan, eprosartan, irbesartan, losartan, telmisartan and valsartan, in particular valsartan; combinantions of NEP inhibitors and angiotensin II antagonists such as sacubitril and valsartan (i.e. Entresto); renin inhibitors such as ditekiren, zankiren, terlakiren, aliskiren, RO 66-1132 and RO-66-1168; beta -adrenergic receptor blockers such as acebutolol, atenolol, betaxolol, bisoprolol, metoprolol, nadolol, propranolol, sotalol and timolol; inotropic agents such as digoxin, dobutamine and milrinone; calcium channel blockers such as amlodipine, bepridil, diltiazem, felodipine, nicardipine, nimodipine, nifedipine, nisoldipine and verapamil; aldosterone receptor antagonists; and aldosterone synthase inhibitors.

The agonist of peroxisome proliferator-activator receptors can be for instance fenofibrate, pioglitazone, rosiglitazone, tesaglitazar, BMS-298585, L-796449, the compounds specifically described in the patent application WO 2004/103995 i.e. compounds of examples 1 to 35 or compounds specifically listed in claim 21, or the compounds specifically described in the patent application WO 03/043985 i.e. compounds of examples 1 to 7 or compounds specifically listed in claim 19 and especially (R)-1-{4-[5-methyl-2-(4-trifluoromethyl-phenyl)-oxazol-4- ylmethoxy]-benzenesulfonyl}-2,3-dihydro-1H-indole-2-carboxylic or a salt thereof.

Other drugs of interest can be for instance cenicriviroc, simtuzumab, selonsertib, emricasan

In a particular embodiment, the one or more additional active drugs used in combination with the peptide can be selected among: a GLP-1 analog such as liraglutide, obeticholic acid, a gliflozin, simtuzumab (GS 6624), cenicriviroc, aramchol, a Galectin 3 inhibitor such as GR-MD-02, a TGR5 agonist and a dual FXR/TGR5 agonist such as INT-777 or INT-767, and emricasan.

The form of the pharmaceutical compositions, the route of administration, the dosage and the regimen naturally depend upon the condition to be treated, the severity of the illness, the age, weight, and sex of the patient, etc.

The pharmaceutical or therapeutic compositions of the present disclosure can be formulated for a topical, oral, parenteral, intranasal, intravenous, intramuscular, subcutaneous or intraocular administration and the like.

The peptide used in the pharmaceutical composition of the present disclosure is present in a therapeutically effective amount.

The pharmaceutical composition comprising the peptide is formulated in accordance with standard pharmaceutical practice (Lippincott Williams & Wilkins, 2000 and Encyclopedia of Pharmaceutical Technology, eds. J. Swarbrick and J. C. Boylan, 1988-1999, Marcel Dekker, New York) known by a person skilled in the art.

In one aspect, the present invention provides a stable formulation for parenteral injection of the pharmaceutical composition according to the present disclosure comprising a peptide or a salt thereof, wherein the peptide has been dried and then is reconstituted in a solvent prior to use. The peptide (or, in embodiments where the formulation comprises two or more peptides, each of the peptides) is mixed with a non-volatile buffer and dried to a dry peptide powder. Suitable buffers include, but are not limited to, glycine buffers, citrate buffers, phosphate buffers, and mixtures thereof. In one embodiment, the buffer is a glycine buffer. In another embodiment, the buffer is a mixture of citrate buffer and phosphate buffer. In some embodiments, wherein the formulation comprises two or more peptides, the first and second buffer are the same. In some embodiments, wherein the formulation comprises two or more peptides, the first and the second buffer are different. Alternatively, the pharmaceutical composition according to the present disclosure may be stored in an aqueous state. The solution may contain, if desired, further additives or excipients, which must be compatible with the active principle and, if they are not removed during the freeze-drying stage, they must also be compatible with the route of administration. For parenteral administration, the composition may be injected intradermally, subcutaneously, intramuscularly, or intravenously. Preferably, the composition or peptide is injected or to be injected subcutaneously, in particular in the fat tissue.

It may preferably be placed with a mini-osmotic pump or other controlled delivery device implanted into the body. Preferably, it may be mixed with other compounds to make a depot slow release formulation. A preferred route of administration is subcutaneous injection, for instance by using a disposable or multiunit dispensing device, similar to an insulin pen.

In one embodiment, the peptide of the present disclosure may be mixed with other compounds to make a depot slow release formulation. This may then be injected subcutaneously to form a slow release depot.

For oral administration, the composition can be formulated into conventional oral dosage forms such as tablets, capsules, powders, granules and liquid preparations such as syrups, elixirs, and concentrated drops. Non-toxic solid carriers or diluents may be used which include, for example, pharmaceutical grades of mannitol, lactose, starch, magnesium stearate, sodium saccharine, talcum, cellulose, glucose, sucrose, magnesium, carbonate, and the like. For compressed tablets, binders, which are agents which impart cohesive qualities to powdered materials, are also necessary. For example, starch, gelatine, sugars such as lactose or dextrose, and natural or synthetic gums can be used as binders. Disintegrants are also necessary in the tablets to facilitate break-up of the tablet. Disintegrants include starches, clays, celluloses, algins, gums and crosslinked polymers. Moreover, lubricants and glidants are also included in the tablets to prevent adhesion to the tablet material to surfaces in the manufacturing process and to improve the flow characteristics of the powder material during manufacture. Colloidal silicon dioxide is most commonly used as a glidant and compounds such as talc or stearic acids are most commonly used as lubricants.

For transdermal administration, the composition can be formulated into ointment, cream or gel form and appropriate penetrants or detergents could be used to facilitate permeation, such as dimethyl sulfoxide, dimethyl acetamide and dimethylformamide.

For transmucosal administration, nasal sprays, intrapulmonary inhalation, rectal or vaginal suppositories can be used. In one embodiment, the invention may be administered by the intrapulmonary route using either a dry powder or liquid formulation administered using an intrapulmonary drug delivery device according to methods known in the art. The active compound can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate.

Pharmaceutical compositions according to the present disclosure may be formulated to release the active drug substantially immediately upon administration or at any predetermined time or time period after administration.

Pharmaceutical compositions according to the present disclosure can comprise one or more peptides of the present disclosure associated with pharmaceutically acceptable excipients and/or carriers. These excipients and/or carriers are chosen according to the form of administration as described above.

In a particular embodiment, the pharmaceutical composition according to the present disclosure comprises between 10 ng and 10 g of the peptide of the present disclosure. In one embodiment, pharmaceutical composition according to the present disclosure comprises between 0.01 mg and 1 g of the peptide of the present disclosure.

All the references cited in this application, including scientific articles and summaries, published patent applications, granted patents or any other reference, are entirely incorporated herein by reference, which includes all the results, tables, figures and texts of theses references.

Although having different meanings, the terms "comprising", "having", "consisting in" and "containing" can be replaced one for the other in the entire application.

Further aspects and advantages of the present disclosure will be described in the following examples, which should be regarded as illustrative and not limiting.

### EXAMPLES

### Example 1: PATAD's long-lasting effect in preventing hyperglycemia in wild type mice

These are results obtained from series of glucose tolerance test (GTT) and data from two time points namely 30 minutes and 120 minutes were used to generate these curves for both control and PATAD treated diet induced obese (DIO) and diabetic wild type male C57/B6 mice (control). These data show the long lasting action of the peptide-based PATAD (3 months post injection of a unique subcutaneous injection).

### Example 2: Effect of adipose tissue targeted PATAD treatment on expression levels of key fatty acids transporters and receptors

As PATAD subcutaneous injection relates to restoration of glucose absorption in the adipose tissue, the inventors measured the expression levels of all 6 isoforms for the FATPs (1-6) (Fatty acid transport protein 1-6) and the 4 isoforms of the FFAR (Free Fatty Acid Receptor) to assess any effect on these genes.

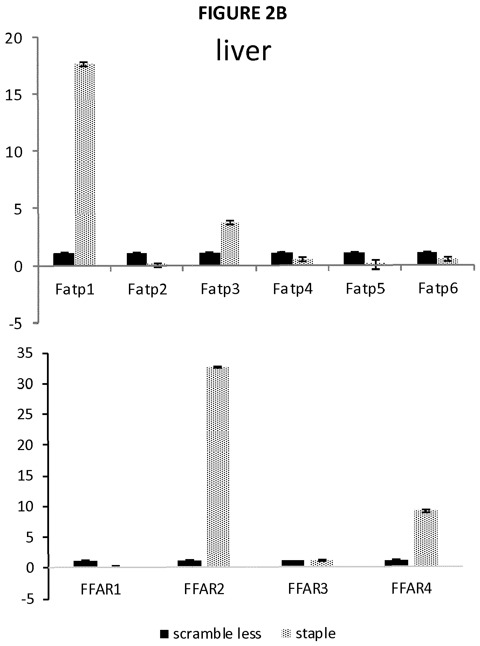

Interestingly, PATAD injections induced a very specific and drastic decrease in FAPT2 in the adipose tissue allowing to correlate the effect of PATAD treatment directly on the FATP2 expression drop (Figure 2A). Since it was previously shown that PATAD peptide does not circulate in the body, that its effect was limited to the adipose tissue and also based on its mechanism of action which is to interfere with ALMS1-PKC interaction, PATAD's novel action in the adipose tissue is to decrease FATP2. The expression levels of the transporters and receptors in the two other major organs can be related to indirect effect (Figure 2B-C).

### Example 3: Protective effect on liver after 3 months of a single injection of adipose tissue targeted PATAD treatment

Mice presented in Figure 1 were euthanized and their plasma and organs were sampled for analysis. The ratio of liver weight to body weight versus age was determined and presented in Figure 3B. The size of the liver in response to the PATAD treatment was clearly decreased compared to controls that were DIO diabetic male controls that received only the vehicle.

Cryosection of the livers were then stained with Adipored and DAPI to detect the level of lipid droplets. PATAD treated mice clearly showed a decrease in the size of the hepatic lipid droplets (Figure 3A, right panel). Hepatic function is assessed by two liver related strong biomarkers namely (AST (Aspartate Aminotransferase) and ALT (Alanine Aminotransferase)), whose levels increase proportionately with liver damage. Interestingly, PATAD treated mice (after a 3-month period with a single subcutaneous PATAD injection) exhibit a dramatic decrease in these two robust hepatic biomarkers (Figure 3C) indicating a protective effect of PATAD on the liver related to a decrease in the phenomenon of steatosis.

### Examples 4 and 5: Circulating lipid profile in response to PATAD treatment in the adipose tissue

PATAD adipose tissue injection has proven to be unexpectedly liver protective relative to a specific decrease in expression levels of FATP2 in the adipose tissue and an associated profile of the FATPs and FFARs in the muscles and liver. As these changes impact circulating population of the different lipids, the inventors measured circulating classical lipids as well as two members of the branched lipids PAHSA with the PAHSA-5 specifically derived from the adipose tissue and known to be a biologically beneficial lipid. They found that there was an associated circulating lipid profile at 3 months post the unique PATAD injection compared to the controls. Of note, they observed an emergence of very long chain ceramides which are known to be protective against the short chain ceramides in the liver for instance. The increase PAHSA-5 is yet another circulating biomarker of the beneficial effect of PATAD adipose tissue injection.

### Materials & Methods

### Mice husbandry

For this study, mice were on a C57/BL6 genetic background. All animals were housed in a temperature and humidity controlled facility, with a 12 h-light/12 h-dark cycle fed during the whole phase mice will be fed with a 60% high fat diet from Research Diets (D12492), and tap water will be provided *ad libitum.* Mice were fed and tested regularly for glucose tolerance test for their glucose tolerance and once they were glucose intolerant they were used for treatment.

### Peptide sequences and synthesis

PATAD is the name given to a series of peptides derived from the PKC alpha isoform that are biologically active with the ability to trigger glucose absorption specifically in the adipose tissue.
Stapled peptide sequence A: VE CTM -[2 - (4-pentenyl) alanine]-EK RVL A-[2 - (4-pentenyl) alanine]-L DKP PFL TQL HS (SEQ ID NO: 19)
Stapled peptide sequence B: S-[2 - (4-pentenyl) alanine]- CKG LMT -[2 - (4-pentenyl) alanine]-HP AKR LGC GPE G (SEQ ID NO: 20)
Scrambled peptide sequence A: KEVPVDTCHLTLMLLFRSVALKQHPE (SEQ ID NO: 21)
Scrambled peptide sequence B: SAECKGRHGTPPGKLMICKGL (SEQ ID NO: 22)

The stapled and scrambled peptides were purchased from Anaspec, USA with a 95% purity.

All peptides were initially dissolved in DMSO and then diluted in sterile saline solution at a concentration of 10ng/µL. 2.5 µL of each peptide (stapled or scrambled) were mixed and then injected directly in the subcutaneaous adipose tissue in each mouse.

### Dosage regimen of the peptides

Control mice was injected with the Scramble controls (retroperitoneal fat/subcutaneous injection: one injection at D0 and the indicated tests performed. Treated mice were injected with the mixture of two PATAD stapled peptides following the same procedure with the PATAD peptide A and B test item (retroperitoneal fat/subcutaneous injection: one injection at D0 and the indicated tests performed.

### RNA extraction, cDNA synthesis, q-PCR and Taqman

Total RNA was prepared from the different tissues and cells using a RiboPure^{™} kit (Catalog #: AM1924; Ambion) followed by a DNAse treatment with the TURBO DNA-free ^{™} (Catalog #: AM 1907; Ambion). RNA integrity was assessed by gel electrophoresis and RNA concentration by Eppendorf Biophotometer Plus with the Hellma® Tray Cell (Catalog #: 105.810-uvs; Hellma). Reverse transcription of 1 µg total RNA to cDNA was performed using the BioRadiScript^{™} cDNA synthesis kit (Catalog #: 170-8891; BioRad). Real-time quantitative polymerase chain reaction amplification was performed in a BioRad CFX96 TM Real-Time System using the iQ^{™} SYBR® Green Supermix (Catalog #: 170-8886; BioRAd) and primer sets optimized for tested targets for SYBR Green-based real-time PCR for the real-time PCR. Taqman analysis was carried out with the specific gene assay with the Taqman® Fast Advanced Master Mix (Catalog #: 4444557; Applied Biosystems). The normalized fold expression of the target gene was calculated using the comparative cycle threshold (Ct) method by normalizing target mRNA Ct to those for GAPDH using the CFX Manager Software Version 1.5 and was verified using the Lin-Reg program (Ruijter et al., 2009). All primer pairs were purchased from Biorad.

Primer specifications are found in the table below:

| Gene name | Primer name | Sequences | SEO ID NO : | Fragment size |
|---|---|---|---|---|
| Fatp1 | Mu_Slc27a1-RT-ex3F | TGCTTTGGTTTCTGGGACTT | 23 | 156bp |
| | Mu_Slc27a1-RT-ex4R | GCTCTAGCCGAACACGAATC | 24 | |
| Fatp2 | Mu_Slc27a2-RT-ex4F | TGGACAAAGTAGACGGAGTGTC | 25 | 165bp |
| | Mu_Slc27a2-RT-ex5R | TAGCAAGGCCTGTCCCATAC | 26 | |
| Fatp3 | Mu_Slc27a3-RT-ex9F | TGAGAACTTGCCACCGTATG | 27 | 171bp |
| | Mu_Slc27a3-RT-ex10R | GGCAGGTAGGCCCCTATATC | 28 | |
| Fatp4 | Mu_Slc27a4-RT-ex2F | GTTTCATCCGGGTCTTCATC | 29 | 184bp |
| | Mu_Slc27a4-RT-ex3R | GTGTCTGTGCCCTCGAAAAT | 30 | |
| Fatp5 | Mu_Slc27a5-RT-ex4F | AAGTTCTCTGCCTCCCGATT | 31 | 191bp |
| | Mu_Slc27a5-RT-ex5R | CAAAGCGTTGCTGGAAGTTT | 32 | |
| Fatp6 | Mu_Slc27a6-RT-ex1F | TCGATTCCCTCCTACACTGC | 33 | 204bp |
| | Mu_Slc27a6-RT-ex2R | TTGGTGGTACTGGCTCATCA | 34 | |

### AdipoRed staining of liver sections

Livers were isolated and briefly washed in PBS buffer (pH 7.4). after weighing the dried liver, for the liver to body weight ratio, a sliced sample of the liver were then placed in 4% paraformaldehyde (in 0.1M sodium phosphate buffer, pH 7.2) for 15 min, washed in PBS and incubated in AdipoRed dye (1/25; Lonza, Switzerland) with 30µM DAPI (Sigma-Aldrich, USA) for 15 minutes. After 3 washes with PBS samples were mounted on slides and pictures were taken using Zeiss microscope.

### AST and ALT elisa measurements

Plasma samples from the indicated mice were used to determine plasma content of either AST (aspartate aminotransferase) or ASL (alanine aminotransferase) both robust indicators of liver damage were measured using commercially purchased ELISA kits. These parameters were determined according to manufacturer's procedure.
- SEB214Mu (96 Tests) : Enzyme-linked Immunosorbent Assay Kit For Aspartate Aminotransferase (AST), Cloud Clone Corp
- SEA207Mu (96 Tests) : Enzyme-linked Immunosorbent Assay Kit For Alanine Aminotransferase (ALT), Cloud Clone Corp

## Claims

1. A peptide for use in the treatment or prevention of a disease selected from the group consisting of nonalcoholic fatty liver disease (NAFLD), non-alcoholic fatty liver (NAFL), non-alcoholic steatohepatitis (NASH), hepatic steatosis (fatty liver), liver fibrosis, liver inflammation, cirrhosis, or hepatocellular carcinoma, wherein
- the peptide modifies the expression levels of the FATPs expression in adipose tissue, preferentially it decreases the FATP2 expression in adipose tissue;
- the peptide comprises the sequence of a segment of a protein kinase C (PKC);
- the peptide comprises at least one methionine, one proline and one arginine ;
- the peptide has a length of at least 8 amino acids and less than 40 amino acids, preferably a length of at least 10 amino acids and less than 30 amino acids; more preferably of at least 12 amino acids and less than 25 amino acids; and
- the peptide may comprise 1, 2, 3, 4, or 5 substitution(s), deletion(s), addition(s), or a mixture thereof within said sequence of a segment of a PKC.

2. The peptide for use according to claim 1, wherein the peptide preferably adopts an alpha helical conformation.

3. The peptide for use according to claim 1 or 2, wherein the peptide is modified by a chemical cross-linking process such as stapling.

4. The peptide for use according to anyone of claims 1-3, wherein the peptide sequence comprises a sequence according to one of the following formulae:
M-(X)ₘ-P-(X)ₙ-R or M-(X)ₘ-R-(X)ₙ-P or P-(X)ₘ-M-(X)ₙ-R or P-(X)ₘ-R-(X)ₙ-M or R-(X)ₘ-P-(X)ₙ-M or R-(X)ₘ-M-(X)ₙ-P
wherein m and n are integers from 1 to 20, m + n is less than 30 and X is any amino acid.

5. The peptide for use according to anyone of claims 1-4, wherein the peptide comprises a sequence according to one of the following formulae:
M-(X)ₘ-R-(X)ₙ-P or P-(X)ₘ-R-(X)ₙ-M or R-(X)ₘ-M-(X)ₙ-P or M-(X)ₘ-P-(X)ₙ-R
wherein m, n and X are as defined in claim 2.

6. The peptide for use according to any one of claims 1-5, wherein m + n is less than 20 and m and n are an integer from 2 to 15, preferably from 2 to 7.

7. The peptide for use according to any one of claims 1-6, wherein said PKC is selected from the group consisting of an alpha-PKC (αPKC), a beta-PKC (βPKC) including βI and βII PKC, delta-PKC, theta-PKC, eta-PKC and epsilon-PKC.

8. The peptide for use according to claim 7, wherein said PKC is an αPKC of SEQ ID No 1.

9. The peptide for use according to any one of claims 1-8, wherein the peptide sequence comprises a sequence selected from the group consisting of:
- MVEKRVLALLDKP (SEQ ID No 2);
- PFLTQLHSCFQTVDRLYFVM (SEQ ID No 3);
- RLYFVMEYVNGGDLMYHIQQVGKFKEP (SEQ ID No 4);
- MYHIQQVGKFKEPQAVFYAAEISIGLFFLHKR (SEQ ID No 5);
- PQAVFYAAEISIGLFFLHKRGIIYRDLKLDNVM (SEQ ID No 6);
- MDGVTTRTFCGTP (SEQ ID No 7); and
- MTKHPAKR (SEQ ID No 8);
and optionally it comprises 1, 2, 3, 4, or 5 substitution(s), deletion(s), addition(s), or a mixture thereof.

10. The peptide for use according to claim 9, wherein the peptide sequence comprises a sequence selected from the group consisting of:
- MVEKRVLALLDKP (SEQ ID No 2);
- MDGVTTRTFCGTP (SEQ ID No 7);
- MTKHPAKR (SEQ ID No 8);
and optionally it comprises 1, 2, 3, 4, or 5 substitution(s), deletion(s), addition(s), or a mixture thereof.

11. The peptide for use according to any one of claims 1-10, wherein the peptide sequence comprises or consists in a sequence selected from the group consisting of:
- VECTMVEKRVLALLDKPPFLTQLHS (SEQ ID No 9);
- MCKEHMMDGVTTRTFCGTPD (SEQ ID No 10);
- SICKGLMTKHPAKRLGCGPEG (SEQ ID No 11);
and optionally it comprises 1, 2, 3, 4, or 5 substitution(s), deletion(s), addition(s), or a mixture thereof.

12. The peptide for use according to anyone of claims 1-11, wherein the peptide consists in a sequence selected from the group consisting of:
- VECTM**V**EKRVLA**L**LDKPPFLTQLHS (SEQ ID No 9);
- MC**K**EHMMDG**V**TTRTFCGTPD (SEQ ID No 10); and
- S**I**CKGLMT**K**HPAKRLGCGPEG (SEQ ID No 11);
wherein the residues which are bold and underlined carry the stapling.

13. The peptide for use according to anyone of claims 1-11, wherein the disease is selected from the group consisting of nonalcoholic fatty liver disease (NAFLD), non-alcoholic fatty liver (NAFL), non-alcoholic steatohepatitis (NASH), and hepatic steatosis (fatty liver).

14. The peptide for use according to anyone of claims 1-11, wherein the disease is hepatic steatosis (fatty liver) or non-alcoholic steatohepatitis (NASH).

15. The peptide for use according to anyone of claims 1-11, wherein the disease is non-alcoholic steatohepatitis (NASH).
